# EUROPEAN PATENT APPLICATION

(11) **EP 4 570 231 A1**
(43) Date of publication of application: **18.06.2025**
(21) Application number: 23397507.7
(22) Date of filing: 13.12.2023
(51) Int. Cl.: A61K 6/30, A61K 6/40, A61K 6/70, A61K 6/77, A61K 6/887

(54) **A KIT OF PARTS FOR DENTISTRY**

(71) Applicant: Stick Tech OY, 20521 Turku (FI)
(72) Inventor: LASSILA, Lippo, 21630 Lielax (FI); GAROUSHI, Sufyan, 20660 Littoinen (FI); VALLITTU, Pekka, 21620 Kuusisto (FI); SÄILYNOJA, Eija, 20660 Littoinen (FI)
(74) Representative: Laine IP Oy

(57) **Abstract**

The present invention relates to a kit of parts for dentistry, comprising a dental primer comprising bis(methacryloyloxyethyl) hydrogen phosphate and 10-methacryloyloxydecyl dihydrogen phosphate; and a dental adhesive.

## Description

### FIELD

The present invention relates to a kit of parts for dentistry, as well as to a use of said kit.

### BACKGROUND AND OBJECTS

Tooth decay, also known as dental caries or cavities, is the breakdown of teeth due to acids made by bacteria. Caries is treated by removing the carious lesions and replacing them with restorative material. Presently the restorative material is mainly based on polymers. These restorative materials have a tendency to shrink, when the monomer is cured to form the polymer, i.e. in situ in the cavity created due to the removal of the carious lesion. Furthermore, the restorative materials may, over time, loosen from the surface of the tooth, which creates cracks and voids, and allows caries to continue destroying the tooth.

The same problem of loosening may also be encountered with crowns and similar, which may become detached from the remaining tooth, and even before complete detachment, allow caries to enter the interface.

Further, minimum intervention dentistry is intending to maintain healthy tooth structure as much as possible. Such approach aims to minimise the excavation of dental tissues and encourage their recovery and repair instead. However, strong and stable bonded interface between the restoration and the tooth hard tissues is the basis for successful dental adhesive restoration. The performance of dentin bonding has been considered more difficult and less predictable than enamel. Dentin bonding relies on the formation of a hybrid layer, which is a structure composed of demineralised collagen fibrils infiltrated by the resin matrix. Up to date, resin-dentin bonds created by infiltration of hydrophilic resin monomers into demineralised dentin matrix are imperfect. Exposed collagen fibrils resulting from the incomplete resin infiltration can be degraded by endogenous and exogenous collagenolytic enzymes. Moreover, these denuded collagen matrices are susceptible to creeping and cracking under mechanical functional challenges.

An aim of the present invention is thus to develop an easy-to-use product for dentistry. Ideally, such product is usable both in preparation of tooth restorations, and in improving attachment of crowns and similar. Advantageously, the product, together with the restoration material, would lead to a strong finished restoration. An aim is thus to achieve durable good bond strength. A further advantage would be that the product would be antimicrobial, i.e. it would limit bacterial growth. A further aim is to provide a material that is able to perform long-lasting ion release to induce dentin remineralisation. A still further aim is to provide a system that is able stop secondary caries formation and regain tough mechanically stabilised structure at the interface to retain tooth's original biomechanical function.

### SUMMARY OF THE INVENTION

The invention is defined by the features of the independent claim. Some specific embodiments are defined in the dependent claims.

According to an aspect, there is provided s kit of parts comprising
- a dental primer comprising bis(methacryloyloxyethyl) hydrogen phosphate and 10-methacryloyloxydecyl dihydrogen phosphate; and
- a dental adhesive.

According to another aspect, there is provided a use of the kit for treating a surface of a dental appliance.

### BRIEF DESCRIPTION OF THE DRAWINGS

Figures 1A and 1B illustrate sample preparation.
Figures 2A and 2B illustrate the test setting for measuring shear-bond strength.
Figures 3 and 4 are scanning electron microscope images of the bond between the dentin and the product of the present kit.
Figure 5 illustrates the results of the marginal gap test.

### DETAILED DESCRIPTION

In the present description, the term "antimicrobial" means that the material kills or inhibits the growth of microorganisms but causes little or no damage to the host. The term "remineralisation of dentin" stands for the natural repair process for non-cavitated tooth lesions, in which calcium, phosphate and sometimes fluoride ions are deposited into crystal voids in demineralised enamel. Remineralisation can contribute towards restoring strength and function within tooth structure. The term "release rate" means the amount of ions released from a sample within a given time.

In the context of the present application, an average length of fibres is determined as follows. The fibres are photographed with a stereo-microscope at a magnification of 6.5 x (or alternatively a scanning electron microscope). The photos are then processed with Image-J processing program to determine the lengths of the fibres. The total number of fibres taken into the calculation is 500. Thereafter, the fibres are divided into a number of fractions with 0.1 mm intervals according to their length. The fibre lengths in each 0.1 mm interval are added together. The average fibre length is taken to be value where the lengths of the shorter fibres and longer fibres are deemed to be the same. This measurement method is described in Lassila et al., "Mechanical properties of fiber reinforced restorative composite with two distinguished fiber length distribution", Journal of the mechanical behavior of biomedical materials 60 (2016) 331-338, section 2.5 on page 333. A diameter of the fibres (i.e. the diameter of a cross-section), if not provided by the manufacturer, can also be determined in this way.

The same determination method can be used for determining an average size of particles, and the measured dimension is the largest dimension of the particle. Most typically, the average particle size of especially the particles is provided by the manufacturer of the particles.

When a concentration of a component in the dental primer is indicated as mg/ml or similar, i.e. weight unit/volume unit, the volume refers to the total volume of the dental primer, even if not always specifically indicated.

According to an aspect of the present invention, there is provided s kit of parts comprising
- a dental primer comprising bis(methacryloyloxyethyl) hydrogen phosphate and 10-methacryloyloxydecyl dihydrogen phosphate; and
- a dental adhesive.

Kit of parts is to be understood as a juxtaposition of separate but functionally interacting individual components, which are combined only when put into use, i.e. the present kit of parts comprises a dental primer comprising bis(methacryloyloxyethyl) hydrogen phosphate and 10-methacryloyloxydecyl dihydrogen phosphate; and a dental adhesive, as separate components configured to functionally interact when used in dentistry. The scope of the claims thus excludes that the listed components are first mixed and then used.

As shown below in the Experimental part, use of the present kit prior to application of a dental restoration decreases the stress at the interface between the dental restoration and the tooth, which increases the clinical lifetime of the restoration. The same applies when the present kit is used to treat the surface of a dental appliance before it is attached to the tooth. Further, as shown below in the Experimental part, the present kit improves mineralisation at demineralised dentin inside dentinal tubules and at the interface between dentin and the present adhesive after two weeks storage in calcium/phosphate rich media. This is believed to contribute to the elimination of secondary caries (i.e. at the caries at the interface), prevent hybrid layer degradation, and increase the longevity of the resin-dentin bond.

The dental primer comprises bis(methacryloyloxyethyl) hydrogen phosphate (bis-MEP), according to an embodiment in a concentration of 0.8-7.5 mg/ml of the dental primer. According to experimental data, concentrations of 0.4 and 8.3 mg/ml still have a small effect, but the range of 0.8-7.5 mg/ml is believed to be the optimum in the present context.

The dental primer comprises 10-methacryloyloxydecyl dihydrogen phosphate (MDP), according to an embodiment in a concentration of 0.8-7.5 mg/ml of the dental primer. According to experimental data, concentrations of 0.4 and 8.3 mg/ml still have a small effect, but the range of 0.8-7.5 mg/ml is believed to be the optimum in the present context. The effect of MDP is that it increases the amount of initial bonds, thus improving the durability of bonded restorations.

According to an embodiment, the concentration of bis-MEP and MDP, independently, is 1.0-6.5 mg/ml. According to another embodiment, the concentration of bis-MEP and MDP, independently, is 2.0-5.5 mg/ml. The concentration of bis-MEP and MDP, independently, may however be for example from 0.8, 0.9, 1.0, 1.2, 1.5, 1.7, 2.0, 2.2, 2.5, 2.7, 3.0, 3.5, 4.0, 4.5, 5.0, 5.5, 6.0, 6.5 or 7.0 mg/ml up to 1.2, 1.5, 1.7, 2.0, 2.2, 2.5, 2.7, 3.0, 3.5, 4.0, 4.5, 5.0, 5.5, 6.0, 6.5, 7.0 or 7.5 mg/ml.

According to a preferred embodiment, the bis-MEP and MDP are present in approximately equal amounts.

According to an embodiment, the dental primer further comprises an ion releasing material in an amount of 0.5-5 wt-% of the total weight of the dental primer. The dental primer may thus comprise an ion releasing material in an amount of from 0.5, 1, 1.5, 2, 2.5, 3, 3.5 or 4 wt-% up to 1.5, 2, 2.5, 3, 3.5, 4, 4.5 or 5 wt-% of the total weight of the dental primer. The ion releasing material enhances remineralisation of dentin. Mineral regeneration in the demineralised dentin is indeed an effective strategy for remineralised dentin to be more resistant to degradation, with improved hardness and elastic modulus.

The ion releasing material may be selected from hydroxyapatite, carbonated apatite, calcium carbonate, calcium phosphate, bioactive glass, zinc oxide containing glass, aluminium calcium silicate glass, titanium oxide containing glass and mixtures thereof. Most preferably the ion releasing material comprises calcium, and typically it is over-saturated with calcium.

According to an embodiment, the ion releasing material comprises calcium and phosphate. According to another embodiment, the ion releasing material comprises calcium and zinc. According to yet another embodiment, the ion releasing material comprises phosphate and zinc. The ion releasing material may also comprise further materials, such as bioactive glass, aluminium and/or titanium.

The release rate of the various ion(s) from the ion releasing material may be controlled in different ways. One way of controlling the release rate is the degree of crystallisation. Typically, if the ion releasing material is an apatite, carbonate and/or glass and highly crystallised (such as having a degree of crystallisation of 20 % or higher, or 70 % or higher, depending on the material), it may be rather inert, i.e. ions are released slowly. The suitable degree of crystallisation for a desired release rate depends on the material selected, and can vary significantly. However, for most materials, for ions to be released quickly, the material should be amorphous. The release rate also depends on the pH of the environment. It is thus possible and advantageous to combine in the ion releasing material at least two different materials having different release rates. In this case, the material with high release rate releases its ions quickly, while the material with lower release rate releases its ions more slowly, and hence over a longer period of time.

According to an embodiment, the dental adhesive comprises reinforcing fibres having a length of 80 µm to 300 µm (micrometre). The reinforcing fibres may be selected from short E-glass fibres, S-glass fibres and mixtures thereof. As shown in the Experimental part below, it is not recommended to include reinforcing fibres in the dental primer, as the thickness of the primer layer is typically smaller than the diameter of reinforcing fibres. The fibres are typically randomly oriented in the finished product. Using short randomly oriented micro-scaled fibres improves the mechanical stability and ability of the adhesive layer at the interface to arrest crack propagation.

More optionally, the average length of the fibres ranges from 100-300 µm. For example, the average length of the filler fibres may be from 100, 120, 150, 170, 200, 220, 230 or 250 µm up to 120, 150, 180, 200, 210, 220, 230, 250, 280 or 300 µm. Optionally, in a preferred embodiment, the average length of the fibres in the material ranges from 150-250 µm.

According to an embodiment, the amount of reinforcing fibres is 10-30 wt-% of the total weight of the dental adhesive. The amount of reinforcing fibres can be for example from 10, 15, 20 or 25 wt-% up to 15, 20, 25 or 30 wt-% of the total weight of the dental adhesive.

The dental adhesive may still further comprise particulate fillers, i.e. filler particles. By particles, it is meant also for example spheres and very short fibres (where the length of the fibre is at most two times its diameter), while being significantly shorter than the short fibres, like whiskers i.e. having a length below 50 µm. The average largest dimension of the particulate fillers can be for example 0.3 - 25 µm. The diameter of the particles in the filler material is thus, in the case of irregular particles, the largest diameter of the particles. The diameter can be from 0.3, 0.5, 1, 5, 7, 10, 13, 15, 17, 20 or 22 µm up to 1, 5, 7, 10, 13, 15, 17, 20, 22 or 25 µm.

Such fillers may be for example bioactive or partially reactive glass ionomer fillers containing elements such as oxides of silicon (Si), calcium (Ca), phosphorus (P), barium (Ba), magnesium (Mg), potassium (K), titanium (Ti), fluorine (F), strontium (Sr), zinc (Zn), cerium (Ce), niobium (Nb) or other compounds of said elements, colour pigments, inert ceramics, inert silica, hydroxyl apatite (HA) or other Ca-phosphates, Al₂O₃, ZrO₂, Ag, zerogels, bioactive glasses or filler particles containing functional bioactive or therapeutically active molecules, antigens, antibiotics, disinfectants, radio-opaque materials, organic acids such as maleic acids, polyacrylic acid, or the like. The therapeutically active molecules may include antimicrobial molecules, or the filler particles may be antimicrobial themselves. Any of these fillers may also be incorporated into the optional dental restoration resin.

According to an embodiment, the kit further comprises a dental restoration material. The dental restoration material is typically a dental restoration material known *per se,* preferably a polymer-based dental restoration material, as it is believed that all known dental restoration materials that mainly consist of a dental restoration resin, polymerisable to a dental restoration material, work with the present invention, and benefit from the use of the present kit, prior to application of the restoration resin.

The dental restoration resin may be for example selected from a group consisting of methyl methacrylate, ethyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diethylene glycol dimethacrylate, triethylene glycol dimethacrylate, diurethane dimethacrylate, 2,2-bis(4-(2-hydroxy-3-methacryloxy)phenyl)propane, acrylic acid, epoxy, bisphenol A-glycidyl methacrylate (BisGMA), urethane dimethacrylate (UDMA), semi-crystalline polyceram (PEX), trimethylolpropane ethoxylate triacrylate and mixtures thereof.

According to another aspect, there is provided a use of the kit for treating a surface of a dental appliance. The dental appliance may be for example a dental crown, overlay, inlay, onlay, veneer and bridge.

The use of the present kit for treating a surface of a dental appliance would increase the adhesion over time of the appliance to the tooth or teeth. As for dental restorations, it would thereby also minimise risks of caries formation underneath the dental applicant. The present kit is suitable for the treatment of various materials of dental appliance's surface, such as metals, ceramics and polymers.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Furthermore, the described features, structures, or characteristics may be combined in any suitable manner in one or more embodiments. In the description, numerous specific details are provided, such as examples of lengths, widths, shapes, etc., to provide a thorough understanding of embodiments of the invention.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### EXPERIMENTAL PART

Several different samples according to the present description were prepared, and compared to samples prepared using commercial products. Various measurements were made, as will be explained below.

The following materials were used in the Examples. When a distribution of length or an average particle size is given, it is the value indicated by the manufacturer.
- E-glass fibres having a diameter of 6 µm and a distribution of length 50-200 µm (i.e. 90 wt-% of the fibres are within this range), from GC Dental
- silica filler: BaAlSiO2 filler particles (diameter 0.7 µm) from Schott (UltraFine GM27884, Schott, Landshut, Germany)
- calcium carbonate anhydrous particles from Sigma-Aldrich
- calcium dihydrogen phosphate hydrate particles from Chempur/PL
- calcium hydrogen phosphate dihydrate particles from Chempur/PL
- tri-calcium phosphate particles from Chempur/PL
- Schott NanoFine 180 particles with an average particle size of 180 nm, from Schott
- niobium particles
- 10-methacryloyloxydecyl dihydrogen phosphate (MDP) from FluoroChem/UK
- bis(methacryloyloxyethyl) hydrogen phosphate (bis-MEP) from Sigma-Aldrich
- BisGMA (bisphenol A-glycidyl methacrylate) from Esstech Inc. (Essington, PA, USA)
- UDMA (urethane dimethacrylate) from Sigma-Aldrich Co. (St Louis, MO, USA)
- HEMA ((hydroxyethyl)methacrylate) from Sigma-Aldrich
- PETA (pentaerythritol tetraacrylate) from Sigma-Aldrich
- camphorquinone (CQ) from Sigma-Aldrich was used as a photoinitiator
- diphenyl iodonium salt (DPI) from Sigma-Aldrich was used a photoinitiator
- ethyl 4-(dimethylamino) benzoate (EDB) from Sigma was used as a photoinitator
- ethanol, AAA-grade
- water, deionised
- G-Premio^{®} Bond from GC Dental (a one-bottle product including a self-etch primer therein)
- Beautifil Flow adhesive from Shofu (flowable, acted as adhesive)
- G-ænial^{®} Universal Injectable from GC Dental
- G-ænial^{®} Achord filling material from GC Dental

The base resin was the same for all the samples, and had the composition shown in Table 1.

**Table 1**

| **Component** | **wt-%** |
|---|---|
| bis-GMA | 28.95 |
| HEMA | 30.48 |
| PETA | 16.93 |
| UDMA | 22.56 |
| CQ | 0.53 |
| EDB | 0.55 |

The dental primer according to the present invention contained the components in amounts given in Table 2, unless otherwise indicated.

**Table 2**

| **Component** | **wt-%** |
|---|---|
| Ethanol | 39.53 |
| Water | 4.74 |
| MDP | 6.59 |
| bis-MEP | 5.01 |
| DPI | 0.66 |
| Base resin | 43.48 |

The fibre reinforced (FRC) adhesive used in the testing contained 25 wt-% of the base resin, 25 wt-% of the E-glass fibres and 50 wt-% of silica as particulate fillers.

### Sample preparation

The following dental primer samples were prepared of the present dental primer. Some samples contained different ion releasing materials. All ion releasing materials were in the form of particles, and were used in the indicated amount in wt-% of the total weight of the primer. Some samples had different amounts of solvents (water and ethanol) to those listed above in Table 2.

When calcium carbonate anhydrous particles, calcium dihydrogen phosphate hydrate particles, calcium hydrogen phosphate dihydrate particles and tri-calcium phosphate particles were added to the samples, it was observed that they were partially dissolved, forming a supernatant liquid, i.e. a supersaturated liquid.
- Sample 1: composition as in Table 2, no ion releasing material; pH was 1-2
- Sample 2: composition as in Table 2, 1.4 wt-% of calcium dihydrogen phosphate hydrate; pH was 1-2
- Sample 3: composition as in Table 2, 1.4 wt-% of calcium hydrogen phosphate dehydrate; pH was 2-4
- Sample 4: composition as in Table 2, 1.4 wt-% of calcium carbonate anhydrous; pH was 6
- Sample 5: composition as in Table 2, 1.4 wt-% of tri-calcium phosphate; pH was 6
- Sample 6: composition as in Table 2, 9 wt-% of Schott's Nanofine
- Sample 7: composition as in Table 2, 1 wt-% of niobium particles
- Sample 8: composition as in Table 2, 1 wt-% of the E-glass fibres
- Sample 9: composition as in Table 2, except that the amount of ethanol was 29.53 wt-%
- Sample 10: composition as in Table 2, except that the amount of ethanol was 49.53 wt-%
- Sample 11: composition as in Table 2, except that the amount of ethanol was 4.74 wt-% and the amount of water 39.53 wt-%; this sample did not adopt the dentin surface, and hence no testing was carried out
- Sample 12: composition as in Table 2, except that no water was used; this sample had so much sediment that it was not tested
- Sample 13: composition as in Table 2, except that the amount of water was 17.5 wt-% (water was added until the mixture was clear)

### Dentin disc preparation

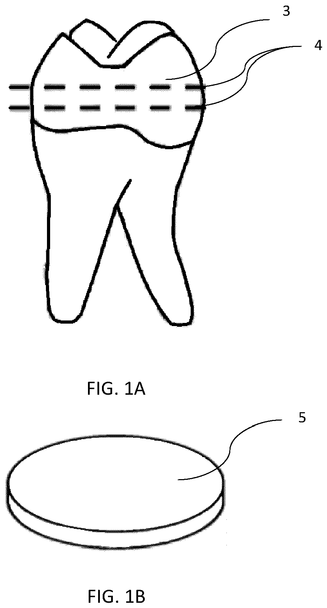

Extracted, sound and caries-free wisdom teeth were selected. Dentin discs (2 mm thick) were prepared a using ceramic cutting disc operating at a speed of 100 rpm (Struers, Glasgow, Scotland) under water cooling. This is illustrated in Figures 1A and 1B. Then, the upper surfaces of dentin discs were polished with an automatic grinding machine, 1200-grit, 300 rpm under running water (Struers Rotopol-11). To simulate demineralized dentin, discs were etched using 37 % phosphoric acid (Scotchbond Universal Etchant, 3M ESPE, USA) at room temperature for 20 s and rinsed carefully with distal distilled water for 1 min.

For testing the interface between the tested experimental adhesive and the demineralised dentin, the present kit (primer and adhesive) and commercial material as a control (G-Premio Bond with G-ænial^{®} Injectable from GC Dental) were applied on discs (n=5/material) and light-cured (Elipar LED) according to the manufacturer's instructions. Then, composite/dentin discs (n=10) were immersed in SBF for 2 weeks (steady) at 37 °C. SBF solution was refreshed every day for 1 week. Discs were then broken into two halves for microscopic evaluation of the interface between the dentin and tested materials.

### Scanning electron microscope

SEM provided the characterization of the microstructure of the investigated specimens. Specimens were stored in desiccator for one day. Then, they were coated with gold using a sputter coater in a vacuum evaporator before the SEM examination.

### Shear bond strength

For the bonding performance measurement, the occlusal surfaces of extracted teeth were wet ground (using an automatic grinding machine, 500-grit, 300 rpm under water cooling; Struers Rotopol-11) to create a flat dentin surface. Subsequently, the teeth were mounted individually into an acrylic block (diameter 2.5 cm) using cold cure auto-polymerised acrylic resin from Palapress; Heraus Kulzer, Wehrheim, Germany

The present primer was applied with or without etching with phosphoric acid (indicated with "Etching yes/no" in Table 3 below) (10 s waiting and then 5 s drying) on a dentin surface and light-cured (Elipar TM S10, 3M ESPE, Germany) for 20 s. Then an adhesive layer was applied and cured for 20 s. For comparison, a commercial material (G-Premio Bond, GC Dental) was used according to manufacturer' instruction. By using a transparent polyethylene mold with an inner diameter of 3.6 mm, bulk increment (2 mm) of conventional composite (G-ænial^{®} Injectable from GC Dental) was applied to the dentin substrates, to form a protrusion having a diameter of 3.6 mm for testing. Composite was photopolymerised twice at full thickness from the side and top surfaces for 20 s using a handheld light-curing unit. The specimens were then stored in water (37 °C) for one day prior to testing.

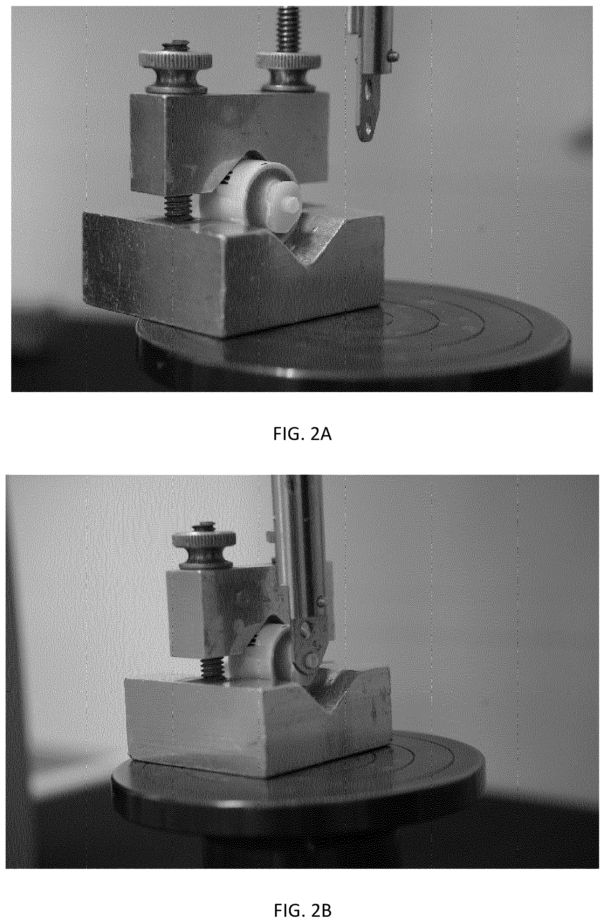

The strength of the bond between the dentin surface and composite layer was measured using a shear bond strength test (Figure 2A and 2B). The specimens were fixed in a mounting jig (Bencor Multi-T shear assembly, Danville Engineering Inc., San Ramon, CA, USA) and a shearing rod was placed parallel to and against the interface. Then, at room temperature (23 ± 1 °C) and a crosshead speed 1.0 mm/min a universal testing machine (Model LRX, Lloyd Instruments Ltd., Fareham, England) was utilized to load the specimens until failure. Data were recorded by PC software (Nexygen, Lloyd Instruments Ltd., Fareham, England). The bond strength was calculated by dividing the maximum load at failure (N) with the bonding area (mm²). The results were recorded in megapascal (MPa).

Failure modes of shear-bond test specimens were visually examined and analysed. The failure modes were then classified either as adhesive failure between composite material and dentin or cohesive type of failure within dentin structure. This gives the cohesive failure-value in percentages, by indicating how the dentine is breaking when the disc is broken.

### Marginal gap test

Class I cavities were prepared in 16 extracted molar teeth. The cavities had a 4-5mm buccal-lingual width, a 5-6 mm mesio-distal length and 4 mm depth. Teeth were distributed into four groups (n=4/group), then cavities were restored according to four different adhesive protocols.

Group A (control): The enamel was acid-etched selectively with 37 % phosphoric acid for 15 s and rinsed with water. After drying the coronal cavity, one-step self-etch adhesive system (G-Premio^{®} Bond) was used, according to the manufacturer's instructions using a microbrush-X disposable applicator. The adhesive was light-cured for 20 s using hand-light curing unit (Elipar TM S10, 3M ESPE, Germany). Then, the cavities were restored with packable conventional composite filling material (G-ænial^{®} Achord, GC Dental) applied and light cured (20 s) in a horizontal layering technique (approximately 2.5 mm thick each).

Group B (present primer without FRC adhesive): The enamel was acid-etched selectively with 37 % phosphoric acid for 15 s and rinsed with water. After drying the coronal cavity, the present primer was applied and light cured for 20 s. Then, the cavities were restored with packable conventional composite filling material as described in Group A.

Group C (present primer with FRC adhesive): Same as group B, but after applying the primer, a layer of FRC adhesive was applied and light cured for 20 s before the application of packable conventional composite filling material as described in Group A.

Group D (present primer with commercial flowable composite, not fiber-reinforced, as adhesive layer): Same as group C but using G-ænial^{®} Universal Injectable (GC Dental) as adhesive in a thin layer instead of using the present FRC adhesive layer.

After restorations, teeth were stored in water (37 °C) for 48 h before the Gap visualisation test.

Teeth were sectioned mid-sagitally in the mesio-distal plane using a ceramic cutting disc operating at a speed of 100 rpm (Struers) under water cooling. In each group, the sectioned restorations (n=8) were ground and polish using #4000-grit silicon carbide papers at 300 rpm under water cooling using an automatic grinding machine (Rotopol-1; Struers). Then, sectioned teeth were painted with a permanent marker, and polished gently for few seconds. The dye penetration along post/core margins of each section was evaluated independently using a stereo-microscope (Heerbrugg M3Z, Heerbrugg) at a magnification of 6.5x and the extent of dye penetration was recorded in mm as a percentage of the total margin length.

### Shear bond strength with cobalt-chromium (Co-Cr) alloy and zirconia

A total of 24 discs (14 mm length × 12 mm width × 3 mm thickness) were prepared and divided into three groups according to the tested substrate. The discs (n=8/group) were positioned in acrylic resin blocks (Palapress Vario; Heraeus Kulzer, Wehrheim, Germany). The surfaces were ground with 180 and 320 grit silicon carbide papers using an automatic grinding machine (Rotopol-1; Struers A/S, Copenhagen Denmark). Then, the surface was air abraded using aluminium oxide particles (Ø 50 µm, pressure 30 psi), followed by washing and air-drying, where after the present primer was applied (sample 1). As a control, one Co-Cr group was treated only by a commercial primer metal (Metal Primer Z, GC) instead of the present primer. Then, custom-made transparent mould with a flat end (diameter 3.6 mm and height 3 mm) was positioned centrally on the flat Co-Cr or zirconia surface. The FRC adhesive was applied and light-cured through the mould on each side and from the top for 20 s (Elipar TM S10). Then, the mould was carefully removed and specimens were stored for 24 h in water (37 °C) before testing. Shear-bond strength test was conducted as explained above.

### Results

### SEM

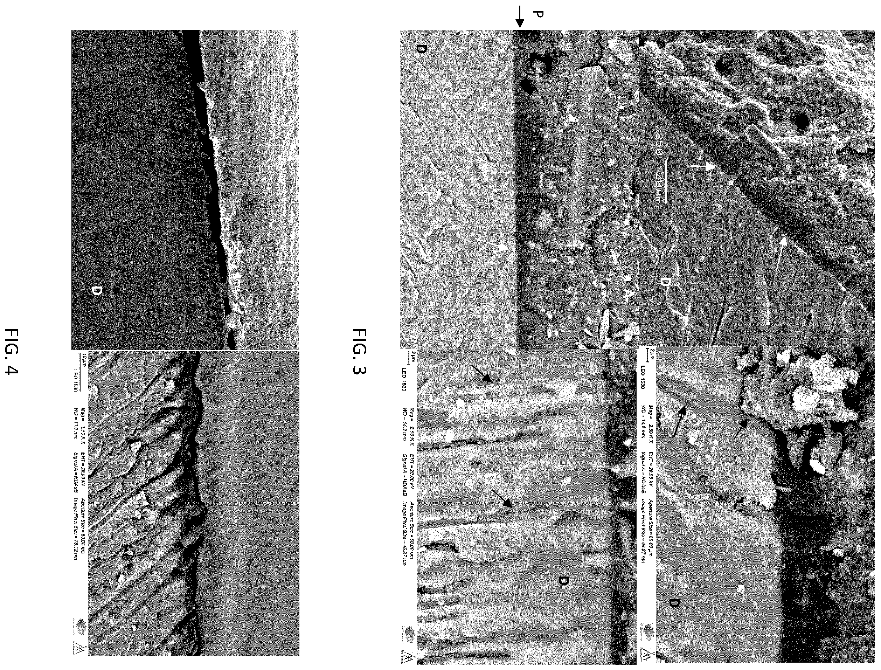

By examining the SEM images, the sealing quality between dentin and the product of the present kit can be observed (Figure 3, white arrows) and evidence of mineralisation at the interface between the fibre reinforced adhesive and dentin and also inside dentinal tubules (Figure 3, black arrows). This is in contrast to the control commercial self-etch bonding system (Figure 4) where there was no sign of mineralisation and there were gaps between the composite and dentin. In both Figures 3 and 4, D stands for dentin and A for adhesive, P stands for primer.

### Shear bond strength and cohesive failure

The results are given below in Tables 3 and 4. The three last lines of Table 3 show comparative examples with a commercial primer. Table 4 shows the results for the shear bond strength with cobalt-chromium (Co-Cr) alloy and zirconia.

As mentioned above, samples 11 and 12 were not tested. It is believed that the results for sample 8 are influenced by the fact that the bonding layer thickness is only 1.2 µm, while the diameter of the E-glass fibres was 6 µm and hence the shear bond strength was not as high as would otherwise be expected.

The results in Table 3 with the different ion releasing materials (samples 2-8) show that while the results vary, they are still all within an acceptable level.

When an adhesive was used, best results were obtained with a fibre reinforced adhesive, where the shear bond strength was better with the present primer than with a commercial primer, used as a comparison.

The results in Table 4 indicate that the present primer demonstrates potential effectiveness also in the shear-bond strength between dental resin and laboratory materials such as cobalt-chromium (Co-Cr) alloy and zirconia.

**Table 3**

| **Dental primer** | **Etching** | **Adhesive** | **Shear bond strength (MPa)** | **Cohesive failure(%)** |
|---|---|---|---|---|
| Sample 1 | yes | | 22.0 | 67 |
| Sample 1 | no | | 20.3 | |
| Sample 2 | yes | | 14.7 | 33 |
| Sample 3 | yes | | 24.7 | 75 |
| Sample 4 | yes | | 20.1 | 22 |
| Sample 5 | yes | | 17.9 | 33 |
| Sample 6 | yes | | 24.3 | 100 |
| Sample 6 | no | | 20.1 | 78 |
| Sample 7 | yes | | 13.2 | |
| Sample 8 | yes | | 15 | |
| Sample 9 | no | | 14.3 | |
| Sample 10 | no | | 14.3 | |
| Sample 13 | no | | 21.3 | |
| Sample 1 | yes | Fibre reinforced adhesive | 20.3 | |
| Sample 1 | yes | Beautifil Flow | 7.6 | |
| Sample 1 | yes | G-ænial^{®} Injectable | 7.7 | |
| G-Premio^{®} Bond | yes | Fibre reinforced adhesive | 16.4 | |
| G-Premio^{®} Bond | yes | Beautifil Flow | 9.1 | |
| G-Premio^{®} Bond | yes | G-ænial^{®} Injectable | 6.6 | |

**Table 4**

| **Dental primer/support** | **Shear bond strength (MPa)** |
|---|---|
| Sample 1 on Co-Cr | 27 MPa |
| Sample 1 on zirconia | 26 MPa |
| Metal Primer on Co-Cr | 31 MPa |

### Marginal gap test

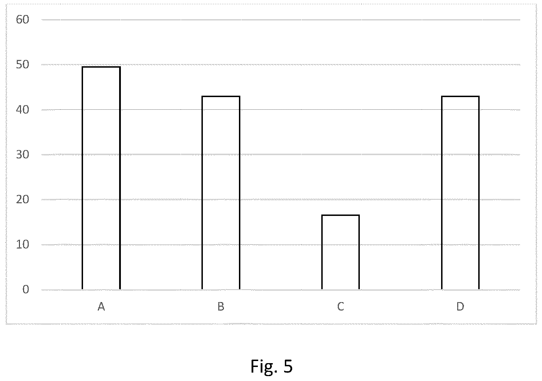

Results are shown in Figure 5 and clearly illustrate that the combination of both present primer and and FRC adhesive layer improved the quality of margins and reduced gaps formation (Group C).

## Claims

1. A kit of parts for dentistry, comprising
- a dental primer comprising bis(methacryloyloxyethyl) hydrogen phosphate and 10-methacryloyloxydecyl dihydrogen phosphate; and
- a dental adhesive.

2. The kit according to claim 1, wherein the dental primer further comprises an ion releasing material in an amount of 0.5-5 wt-% of the total weight of the dental primer.

3. The kit according to claim 3, wherein the ion releasing material is selected from hydroxyapatite, carbonated apatite, calcium carbonate, calcium phosphate, bioactive glass, zinc oxide containing glass, aluminium calcium silicate glass, titanium oxide containing glass and mixtures thereof.

4. The kit according to any of the preceding claims, wherein the dental adhesive comprises reinforcing fibres having a length of 80 µm to 300 µm.

5. The kit according to claim 4, wherein the reinforcing fibres are selected from short E-glass fibres, S-glass fibres and mixtures thereof.

6. The kit according to claim 4 or 5, wherein the amount of reinforcing fibres is 10-30 wt-% of the total weight of the dental adhesive.

7. The kit according to any of the preceding claims, wherein the concentration of 10-methacryloyloxydecyl dihydrogen phosphate in the dental primer is 0.8-7.5 mg/ml.

8. The kit according to any of the preceding claims, further comprising a dental restoration material.

9. The kit according to claim 8, wherein the dental restoration resin is selected from a group consisting of methyl methacrylate, ethyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diethylene glycol dimethacrylate, triethylene glycol dimethacrylate, diurethane dimethacrylate, 2,2-bis(4-(2-hydroxy-3-methacryloxy)phenyl)propane, acrylic acid, epoxy, bisphenol A-glycidyl methacrylate (BisGMA), urethane dimethacrylate (UDMA), semi-crystalline polyceram (PEX), trimethylolpropane ethoxylate triacrylate and mixtures thereof.

10. Use of a kit according to any of the claims 1-9 for treating a surface of a dental appliance.
